# EUROPEAN PATENT APPLICATION

(11) **EP 4 530 280 A1**
(43) Date of publication of application: **02.04.2025**
(21) Application number: 22943847.8
(22) Date of filing: 19.12.2022
(51) Int. Cl.: C07C 31/20, A61Q 19/00, A61K 8/34

(54) **1,3-BUTYLENE GLYCOL PRODUCT**

(30) Priority: 23.05.2022 JP 2022083950
(71) Applicant: KH Neochem Co., Ltd., Tokyo 103-0022 (JP)
(72) Inventor: MAEDA, Miki, Yokkaichi-shi, Mie 510-8502 (JP); HAKUMURA, Takashi, Yokkaichi-shi, Mie 510-8502 (JP); KANADA, Jun, Yokkaichi-shi, Mie 510-8502 (JP)
(74) Representative: V.O.
(86) International application number: PCT/JP2022/046577
(87) International publication number: WO 2023/228448

(57) **Abstract**

A product 1,3-butylene glycol, in which, in a gas chromatography analysis by a splitless injection method under specific conditions, when a relative retention time of n-dodecane is regarded as 1.00, a sum of n-dodecane equivalent concentrations of peaks that appear within a relative retention time range of 0.80 to 0.99, excluding a peak of an acetal form between methyl ethyl ketone and 1,3-butylene glycol, is 25 ppm or less.

## Description

### Technical Field

The present invention relates to a product 1,3-butylene glycol that is useful as a raw material of synthetic resins, a raw material of surfactants, a solvent, an antifreeze, a cosmetic raw material or the like.

### Background Art

1,3-Butylene glycol is a viscous, colorless and transparent liquid having a low odor and a boiling point of 208°C and has excellent chemical stability.

Therefore, 1,3-butylene glycol is used as a raw material of a variety of synthetic resins and surfactants. 1,3-Butylene glycol is also in use as a material for cosmetics, moisture absorbers, high-boiling solvents and antifreezes due to its excellent moisture absorption characteristic, low volatility and low toxicity. Particularly, in recent years, the demand for 1,3-butylene glycol has been significantly growing in the cosmetic industry since 1,3-butylene glycol having low toxicity and low stimulus has excellent properties as a moisturizer.

Patent Literature 1 discloses 1,3-butylene glycol having a weak odor and discloses, as a method for obtaining 1,3-butylene glycol having a weak odor, a production method for 1,3-butylene glycol in which crude 1,3-butylene glycol is purified by a hydrogenation treatment.

### Citation List

### Patent Literature

Patent Literature 1: Japanese Translation of PCT International Application Publication No. 2020-512351

### Summary of Invention

### Technical Problem

For example, in the cosmetics field, 1,3-butylene glycol is directly applied to the skin during use, but 1,3-butylene glycol that is obtained by the method described in Patent Literature 1 has a problem in that skin sensitization has not yet been sufficiently reduced. Furthermore, in the cosmetics field, there are cases where 1,3-butylene glycol is heated and blended under a basic condition during use, but there is another problem in that 1,3-butylene glycol colors when mixed and prepared under a basic condition.

In consideration of the above-described circumstances, an objective of the present invention is to provide a product 1,3-butylene glycol that causes less skin sensitization and also does not easily color under a basic condition.

### Solution to Problem

As a result of intensive studies, the present inventors have found that the above-described problems can be solved by suppressing the concentration of specific impurities that are contained in 1,3-butylene glycol to a certain level or lower and have completed the present invention.

More specifically, the present invention is as described below. In the present specification, ppm indicates mass ppm unless particularly otherwise described.
[1] A product 1,3-butylene glycol, wherein, in a gas chromatography analysis by a splitless injection method under the following conditions, when a relative retention time of n-dodecane is regarded as 1.00, a sum of n-dodecane equivalent concentrations of peaks that appear within a relative retention time range of 0.80 to 0.99, excluding a peak of an acetal form between methyl ethyl ketone and 1,3-butylene glycol, is 25 ppm or less,
   wherein the conditions of the gas chromatography analysis are as follows:
   analysis column: a column in which a stationary phase is (50% cyanopropyl-phenyl) dimethylpolysiloxane (length 30 m × inner diameter 0.25 mm × film thickness 0.25 µm),
   temperature rising conditions: a temperature is retained at 50°C for five minutes, then, raised from 50°C up to 135°C at 10 °C/minute, retained at 135°C for nine minutes, raised from 135°C up to 220°C at 15 °C/minute and retained at 220°C for 12 minutes,
   sample introduction temperature: 220°C,
   carrier gas: nitrogen,
   gas flow rate in column: 0.5 mL/minute,
   detector and detection temperature: flame ionization detector (FID) and 220°C,
   control mode: constant flow,
   split ratio: splitless,
   injection opening vent purge: 60 mL/minute,
   purge start time: one minute,
   sample injection condition: 0.6 µL,
   sample preparation: n-dodecane, which is an inner standard substance, is diluted with 2-propanol to 24 ppm to prepare an inner standard solution, and then 0.5 g of the product 1,3-butylene glycol and 0.1 g of the inner standard solution are mixed together, thereby preparing a sample,
   calculation of sum of n-dodecane equivalent concentrations: in a case where the relative retention time of n-dodecane that is detected under the gas chromatography analysis conditions is regarded as 1.00, a total area value of peaks that are detected within the relative retention time range of 0.80 to 0.99, excluding the peak of the acetal form between methyl ethyl ketone and 1,3-butylene glycol (a detected peak area value) is converted to the sum of the n-dodecane equivalent concentrations using the following formula (1), the sum of n-dodecane equivalent concentrations (ppm) = an n-dodecane concentration (ppm) × the detected peak area value/an area value of n-dodecane (1),
   here, the n-dodecane concentration (ppm) is a value that is calculated using the following formula (2), the n-dodecane concentration (ppm) = (a mass of n-dodecane in sample/a mass of product 1,3-butylene glycol in sample) × 106 (2).
[2] A product 1,3-butylene glycol, wherein, in a gas chromatography analysis by a splitless injection method under the following conditions, a sum of n-dodecane equivalent concentrations of a peak of 2-ethylcrotonaldehyde and a peak of an acetal form between n-butyraldehyde and 1,3-butylene glycol is 20 ppm or less,
   wherein the conditions of the gas chromatography analysis are as follows:
   analysis column: a column in which a stationary phase is (50% cyanopropyl-phenyl) dimethylpolysiloxane (length 30 m × inner diameter 0.25 mm × film thickness 0.25 µm),
   temperature rising conditions: a temperature is retained at 50°C for five minutes, then, raised from 50°C up to 135° at 10 °C/minute, retained at 135°C for nine minutes, raised from 135°C up to 220°C at 15 °C/minute and retained at 220°C for 12 minutes,
   sample introduction temperature: 220°C,
   carrier gas: nitrogen,
   gas flow rate in column: 0.5 mL/minute,
   detector and detection temperature: flame ionization detector (FID) and 220°C,
   control mode: constant flow,
   split ratio: splitless,
   injection opening vent purge: 60 mL/minute,
   purge start time: one minute,
   sample injection condition: 0.6 µL,
   sample preparation: n-dodecane, which is an inner standard substance, is diluted with 2-propanol to 24 ppm to prepare an inner standard solution, and then 0.5 g of the product 1,3-butylene glycol and 0.1 g of the inner standard solution are mixed together, thereby preparing a sample,
   calculation of sum of n-dodecane equivalent concentrations: a total area value of the peak of 2-ethylcrotonaldehyde and the peak of the acetal form between n-butyraldehyde and 1,3-butylene glycol that are detected under the gas chromatography analysis condition (a detected peak area value) is converted to the sum of the n-dodecane equivalent concentrations using the following formula (1), the sum of n-dodecane equivalent concentrations (ppm) = an n-dodecane concentration (ppm) × the detected peak area value/an area value of n-dodecane (1),
   here, the n-dodecane concentration (ppm) is a value that is calculated using the following formula (2), the n-dodecane concentration (ppm) = (a mass of n-dodecane in sample/a mass of product 1,3-butylene glycol in sample) × 106 (2).
[3] The product 1,3-butylene glycol according to [1] or [2], wherein, in the gas chromatography analysis under the following conditions, an area percentage of a peak of 1,3-butylene glycol is 99.5% or more,
   wherein the conditions of the gas chromatography analysis are as follows:
   analysis column: a column in which a stationary phase is polyethylene glycol (length 30 m × inner diameter 0.25 mm × film thickness 0.25 µm),
   temperature rising conditions: the temperature was raised from 80°C up to 230°C at 5 °C/minute and then retained at 230°C for 10 minutes,
   sample introduction temperature: 250°C,
   carrier gas: nitrogen,
   gas flow rate in column: 0.5 mL/minute,
   detector and detection temperature: flame ionization detector (FID) and 250°C,
   control mode: constant flow,
   split ratio: 50:1,
   sample injection condition: 1 µL.

### Advantageous Effects of Invention

The present invention makes it possible to provide a product 1,3-butylene glycol that causes less skin sensitization and also does not easily color under a basic condition.

### Description of Embodiments

Hereinafter, an embodiment of the present invention (hereinafter, referred to as "present embodiment") will be described in detail. The present invention is not limited to the following description and can be carried out after being modified in a variety of manners within the scope of the gist thereof.

In the present embodiment, 1,3-butylene glycol that is the final product will be also referred to as "product 1,3-butylene glycol", and 1,3-butylene glycol as a raw material or an intermediate formed before the final product will be also referred to as "crude 1,3-butylene glycol".

[Production Method for Product 1,3-Butylene Glycol]

### (Raw Material)

Crude 1,3-butylene glycol that is used as a raw material in producing the product 1,3-butylene glycol in the present embodiment is not particularly limited, and examples thereof include 1,3-butylene glycol causing skin sensitization or 1,3-butylene glycol that colors under a basic condition.

As the crude 1,3-butylene glycol as the raw material, the area percentage of the peaks of 1,3-butylene glycol in a gas chromatography analysis 2 under specific conditions to be described below is preferably 99.5% or more, more preferably 99.6% or more and still more preferably 99.7% or more from the viewpoint of reducing the amount of impurities that are contained in the product 1,3-butylene glycol.

A production method for the crude 1,3-butylene glycol as the raw material is not particularly limited, and the crude 1,3-butylene glycol can be produced by, for example, a well-known method (refer to Japanese Patent Publication No. H3-80139, Japanese Patent Laid-Open No. 7-258129 and the like). In addition, any of a product from acetaldol by a liquid phase hydrogen reduction method, a product from 1,3-butylene oxide by a hydrolysis method, a product by a fermentation method in which a microbe or a fungus is used, a mixture thereof and the like may also be used. Among these, a reaction product obtained from acetaldol by the liquid phase hydrogen reduction method is preferably used since the effect of the present invention tends to become more significant. In the method of liquid phase hydrogen reduction of acetaldol, low-boiling compounds such as acetaldehyde, n-butyraldehyde, crotonaldehyde and methyl vinyl ketone, which are considered to be odor-causing substances, a condensation product between them, or acetals between them and 1,3-butylene glycol, acetals between them and ethanol, and the like are generated as byproducts, and it is difficult to sufficiently remove low-boiling compounds and the like even by distillation. There is another problem in that, even in a heating step such as distillation, 2-ethylcrotonaldehyde or the like as a byproduct is generated by a condensation reaction of acetaldehyde with n-butyraldehyde, or an acetal form between n-butyraldehyde and 1,3-butylene glycol is generated as a byproduct. The odor-causing substance includes a substance that acts as an odor source, and a substance that turns into an odor substance by a change over time, a heat treatment, a chemical treatment or the like.

The reaction product that is obtained by the method of liquid phase hydrogen reduction of acetaldol may also be used as the crude 1,3-butylene glycol after alcohols such as ethanol, salts, moisture and the like, which are byproducts, are removed. A method for removing the above-described components is not limited, and a method such as distillation or adsorption can be used.

A substance obtained by further performing one or more well-known purification steps for a distillate from which ethanol and the like have been removed, for example, steps of adding an alkali metal compound (for example, sodium hydroxide, potassium hydroxide or the like) thereto and performing a heat treatment (refer to Japanese Patent No. 4559625 or the like) may also be used as the crude 1,3-butylene glycol. The crude 1,3-butylene glycol can also be obtained as a commercially available product.

### (Hydrogenation Treatment Step)

The production method for the product 1,3-butylene glycol in the present embodiment includes a step of performing a hydrogenation treatment on the crude 1,3-butylene glycol using a catalyst (hydrogenation treatment step). The hydrogenation treatment step is more specifically, for example, a step of performing hydrogen reduction by circulating the crude 1,3-butylene glycol and a hydrogen gas in a catalyst layer packed with a catalyst.

When the hydrogenation treatment is performed on the crude 1,3-butylene glycol using a catalyst, it is presumed that 2-ethylcrotonaldehyde and an acetal form between n-butyraldehyde and 1,3-butylene glycol, which are substances causing coloration under a basic condition and skin sensitization, are hydrogenated or hydrocracked. However, the mechanism of the present invention is not limited to what has been described above.

The hydrogenation treatment step can be performed in any of a gas phase or a liquid phase and is preferably performed in a liquid phase. In a case where the hydrogenation treatment step is performed in a liquid phase, since it is possible to set the reaction temperature to a low temperature compared with the case of being performed in a gas phase, a pyrolytic reaction of 1,3-butylene glycol can be suppressed. In the hydrogenation treatment step, it is preferable to use a Pd/C catalyst as the catalyst. The Pd/C catalyst refers to a catalyst containing palladium dispersed and carried in activated carbon as a carrier. The carrying percentage of palladium dispersed and carried in activated carbon is not particularly limited, but is preferably 0.1% to 30%, more preferably 0.2% to 10% and particularly preferably 0.4% to 1%. The shape of the Pd/C catalyst is not particularly limited and may be a shape of a powder, a pellet or the like.

The reaction temperature in the hydrogenation treatment step is not particularly limited; however, in a case where a heat treatment step to be described below is performed, the reaction temperature is preferably 100°C to 150°C, more preferably 110°C to 145°C and still more preferably 125°C to 135°C, and, in a case where the heat treatment step to be described below is not performed, the reaction temperature is preferably higher than 120°C to 150°C and more preferably 125°C to 135°C.

When the reaction temperature in the hydrogenation treatment step is 150°C or lower, there is a tendency that the pyrolytic reaction of 1,3-butylene glycol does not progress and an increase in high-boiling components is suppressed, and, when the reaction temperature is 100°C or higher (in a case where the heat treatment step is not performed, higher than 120°C), the progress of the hydrogenation or hydrocracking of 2-ethylcrotonaldehyde and the acetal form between n-butyraldehyde and 1,3-butylene glycol tends to be accelerated.

The hydrogen pressure in the hydrogenation treatment step is not particularly limited, but is preferably 0.4 to 1.0 MPa, more preferably 0.5 to 0.9 MPa and still more preferably 0.6 to 0.8 MPa. When the hydrogen pressure in the hydrogenation treatment step is 1.0 MPa or lower, the device cost tends to be reduced since a thick-walled device or the like having a sufficient strength is not required as a high-pressure gas facility, and, when the hydrogen pressure is 0.4 MPa or higher, the progress of the hydrogenation or hydrocracking of 2-ethylcrotonaldehyde and the acetal form between n-butyraldehyde and 1,3-butylene glycol tends to be accelerated.

The production method for the product 1,3-butylene glycol in the present embodiment is not particularly limited as long as the step of performing the hydrogenation treatment on the crude 1,3-butylene glycol using the catalyst (hydrogenation treatment step) is included, and one or more of a step of heat-treating the crude 1,3-butylene glycol (heat treatment step) and a step of distilling low-boiling components away from the crude 1,3-butylene glycol (low-boiling fraction distillation step) may be further included in addition to the hydrogenation treatment step. The order of these steps is not particularly limited; however, from the viewpoint of the effect of the invention of the present application becoming more significant, it is preferable to perform the heat treatment step, the hydrogenation treatment step and the low-boiling fraction distillation step in order.

Hereinafter, each step will be described.

### (Heating Treatment Step)

The heat treatment step in the production method for the product 1,3-butylene glycol of the present embodiment is a step of heat-treating the crude 1,3-butylene glycol. When the crude 1,3-butylene glycol is heat-treated, acetaldehyde and n-butyraldehyde, which are causative substances that generate 2-ethylcrotonaldehyde as a byproduct, are consumed due to a condensation reaction or the like, or n-butyraldehyde, which is a causative substance that generates the acetal form between n-butyraldehyde and 1,3-butylene glycol as a byproduct, is consumed, and therefore in a step involving heating such as the subsequent distillation step, the amount of 2-ethylcrotonaldehyde and the acetal form between n-butyraldehyde and 1,3-butylene glycol generated is reduced. When 2-ethylcrotonaldehyde, the acetal form between n-butyraldehyde and 1,3-butylene glycol or the like is generated as a byproduct in advance in the heat treatment step and then the hydrogenation treatment is performed, the causative substances tend to be more efficiently decomposed by hydrogenation or hydrocracking. However, the mechanism of the present invention is not limited to what has been described above.

The heating time in the heat treatment step is not particularly limited, but is preferably 20 minutes to nine hours, more preferably one to six hours and still more preferably one to three hours. When the heating time is 20 minutes or longer, the consumption of acetaldehyde and n-butyraldehyde by a condensation reaction sufficiently tends to progress, and when the heating time is nine hours or shorter, an increase in the cost taken for the heat treatment tends to be suppressible.

The heating temperature in the heat treatment step is not particularly limited, but is preferably 80°C to 200°C, more preferably 90°C to 120°C and still more preferably 100°C to 110°C. When the heating temperature is 80°C or higher, the condensation reaction of acetaldehyde and n-butyraldehyde tends to progress, and when the heating temperature is 200°C or lower, there is a tendency that a hydrolysis reaction of 1,3-butylene glycol is suppressed and impurities are reduced.

A heat treatment device in the heat treatment step is not particularly limited, and examples thereof include heat treatment devices such as a continuous tube-type device, a batch tank-type device and a continuous tank-type device, and the batch tank-type device is particularly preferable from the viewpoint of the stirring efficiency.

### (Low-Boiling Fraction Distillation Step)

The low-boiling fraction distillation step in the production method for the product 1,3-butylene glycol of the present embodiment is a step of distilling the low-boiling components away from, for example, a distillate containing a large amount of 1,3-butylene glycol obtained in the hydrogenation treatment step. Examples of a distillation device that is used in the low-boiling fraction distillation step include a perforated plate tower, a bubble cap tower and a packed tower, and, among these, a packed tower having seven to 40 theoretical plates is preferable. The distillation tower may be one tower or two or more towers may be used. As the distillation conditions, the pressure at the tower top portion of the distillation tower is preferably 1 to 20 kPa, and the temperature at the tower bottom portion of the distillation tower is preferably 100°C to 160°C and more preferably 110°C to 140°C. A specific aspect of the low-boiling fraction distillation step may be a continuous method or a batch method, and examples of the continuous method include a method in which a distillate containing a large amount of 1,3-butylene glycol is continuously supplied from the tower top of the distillation tower, a distillate containing a large amount of the low-boiling components is continuously extracted from the tower top and, simultaneously, the distillate containing a larger amount of 1,3-butylene glycol is continuously extracted from the tower bottom.

### [Product 1,3-Butylene Glycol]

As one of the present embodiment, provided is a product 1,3-butylene glycol, in which, in a gas chromatography analysis by a splitless injection method under the following conditions, when the relative retention time of n-dodecane is regarded as 1.00, the sum of n-dodecane equivalent concentrations of peaks that appear within a relative retention time range of 0.80 to 0.99, excluding the peak of an acetal form between methyl ethyl ketone and 1,3-butylene glycol, is 25 ppm or less.

### [Conditions of Gas Chromatography Analysis]

Analysis column: A column in which the stationary phase is (50% cyanopropyl-phenyl) dimethylpolysiloxane (length 30 m × inner diameter 0.25 mm × film thickness 0.25 µm)
Temperature rising conditions: The temperature is retained at 50°C for five minutes, then, raised from 50°C up to 135°C at 10 °C/minute, retained at 135°C for nine minutes, raised from 135°C up to 220°C at 15 °C/minute and retained at 220°C for 12 minutes.
Sample introduction temperature: 220°C
Carrier gas: Nitrogen
Gas flow rate in column: 0.5 mL/minute
Detector and detection temperature: Flame ionization detector (FID) and 220°C
Control mode: Constant flow
Split ratio: Splitless
Injection opening vent purge: 60 mL/minute
Purge start time: One minute
Sample injection condition: 0.6 µL

Sample preparation: n-Dodecane, which is an inner standard substance, is diluted with 2-propanol to 24 ppm to prepare an inner standard solution, and then 0.5 g of the product 1,3-butylene glycol and 0.1 g of the inner standard solution are mixed together, thereby preparing a sample.

Calculation of sum of n-dodecane equivalent concentrations: In a case where the relative retention time of n-dodecane that is detected under the gas chromatography analysis conditions is regarded as 1.00, the total area value of peaks that are detected within the relative retention time range of 0.80 to 0.99, excluding the peak of the acetal form between methyl ethyl ketone and 1,3-butylene glycol (the detected peak area value) is converted to the sum of the n-dodecane equivalent concentrations using the following formula (1). The sum of n-dodecane equivalent concentrations (ppm) = the n-dodecane concentration (ppm) × the detected peak area value/the area value of n-dodecane (1)

Here, the n-dodecane concentration (ppm) is a value that is calculated using the following formula (2). The n-dodecane concentration (ppm) = (the mass of n-dodecane in sample/the mass of product 1,3-butylene glycol in sample) × 106 (2)

Here, as the analysis column, for example, DB-225 (length 30 m × inner diameter 0.25 mm × film thickness 0.25 µm, stationary phase: (50% cyanopropyl-phenyl) dimethylpolysiloxane) manufactured by Agilent Technologies, Inc. can be used.

In the product 1,3-butylene glycol, which is one of the present embodiment, the sum of n-dodecane equivalent concentrations of peaks that appear within a relative retention time range of 0.80 to 0.99, excluding the peak of the acetal form between methyl ethyl ketone and 1,3-butylene glycol, is 25 ppm or less, preferably 23 ppm or less and more preferably 21 ppm or less because the effect exhibited by the invention of the present application tends to become more significant.

As one of the present embodiment, provided is a product 1,3-butylene glycol, in which, in a gas chromatography analysis by a splitless injection method under the following conditions, the sum of n-dodecane equivalent concentrations of the peak of 2-ethylcrotonaldehyde and the peak of an acetal form between n-butyraldehyde and 1,3-butylene glycol is 20 ppm or less.

### [Conditions of Gas Chromatography Analysis]

Analysis column: A column in which the stationary phase is (50% cyanopropyl-phenyl) dimethylpolysiloxane (length 30 m × inner diameter 0.25 mm × film thickness 0.25 µm)
Temperature rising conditions: The temperature is retained at 50°C for five minutes, then, raised from 50°C up to 135°C at 10 °C/minute, retained at 135°C for nine minutes, raised from 135°C up to 220°C at 15 °C/minute and retained at 220°C for 12 minutes.
Sample introduction temperature: 220°C
Carrier gas: Nitrogen
Gas flow rate in column: 0.5 mL/minute
Detector and detection temperature: Flame ionization detector (FID) and 220°C
Control mode: Constant flow
Split ratio: Splitless
Injection opening vent purge: 60 mL/minute
Purge start time: One minute
Sample injection condition: 0.6 µL
Sample preparation: n-Dodecane, which is an inner standard substance, is diluted with 2-propanol to 24 ppm to prepare an inner standard solution, and then 0.5 g of the product 1,3-butylene glycol and 0.1 g of the inner standard solution are mixed together, thereby preparing a sample.
Calculation of sum of n-dodecane equivalent concentrations: The total area value of the peak of 2-ethylcrotonaldehyde and the peak of the acetal form between n-butyraldehyde and 1,3-butylene glycol that are detected under the gas chromatography analysis condition (the detected peak area value) is converted to the sum of the n-dodecane equivalent concentrations using the following formula (1). The sum of n-dodecane equivalent concentrations (ppm) = the n-dodecane concentration (ppm) × the detected peak area value/the area value of n-dodecane (1)
Here, the n-dodecane concentration (ppm) is a value that is calculated using the following formula (2). The n-dodecane concentration (ppm) = (the mass of n-dodecane in sample/the mass of product 1,3-butylene glycol in sample) × 106 (2)

Here, as the analysis column, for example, DB-225 (length 30 m × inner diameter 0.25 mm × film thickness 0.25 µm, stationary phase: (50% cyanopropyl-phenyl) dimethylpolysiloxane) manufactured by Agilent Technologies, Inc. can be used.

In the product 1,3-butylene glycol, which is one of the present embodiment, the sum of n-dodecane equivalent concentrations of the peak of 2-ethylcrotonaldehyde and the peak of the acetal form between n-butyraldehyde and 1,3-butylene glycol is 20 ppm or less, preferably 16 ppm or less and more preferably 12 ppm or less because the effect exhibited by the invention of the present application tends to become more significant.

In the product 1,3-butylene glycol of the present embodiment, the area percentage of the peaks of 1,3-butylene glycol in the gas chromatography analysis under the following conditions is preferably 99.5% or more, more preferably 99.7% or more, still more preferably 99.8% or more and particularly preferably 99.9% or more depending on required product qualities. The "area percentage" of peaks means the proportion of the area of a specific peak in the sum of the areas of all peaks that appear in a chart. All peaks mean all peaks that appear in a case where the analysis is continued until the relative retention time reaches 2.2 and stopped when the relative retention time of the peak of 1,3-butylene glycol is regarded as 1.0. When the area percentage of the peaks is within the above-described range, the generation of an odor or skin sensitization tends to be further reduced.

### [Conditions of Gas Chromatography Analysis]

Analysis column: A column in which the stationary phase is polyethylene glycol (length 30 m × inner diameter 0.25 mm × film thickness 0.25 µm)
Temperature rising conditions: The temperature is raised from 80°C up to 230°C at 5 °C/minute and then retained at 230°C for 10 minutes.
Sample introduction temperature: 250°C
Carrier gas: Nitrogen
Gas flow rate in column: 0.5 mL/minute
Detector and detection temperature: Flame ionization detector (FID) and 250°C
Control mode: Constant flow
Split ratio: 50:1
Sample injection condition: 1 µL

Here, as the analysis column, for example, DB-WAX (column in which the stationary phase is polyethylene glycol; length 30 m × inner diameter 0.25 mm × film thickness 0.25 µm) manufactured by Agilent Technologies, Inc. can be used.

### [Skin Sensitization Test]

The product 1,3-butylene glycol in the present embodiment causes less skin sensitization. Here, the skin sensitization refers to the occurrence of an allergic reaction after the product 1,3-butylene glycol comes into contact with the skin. In the evaluation of skin sensitization, it has been normal to use experimental animals, but the direct peptide reactivity assay (DPRA), which is an in Chemico test, was adopted as an alternative test in OECD guideline TG442C in 2015 from the viewpoint of animal welfare, and, in the present embodiment as well, the skin sensitization is evaluated by a test in which DPRA is used. In more detail, the skin sensitization is evaluated according to a method described in examples to be described below.

### [Coloration under Basic Condition]

The product 1,3-butylene glycol in the present embodiment has an advantage of not easily coloring under a basic condition. Coloration under a basic condition can be evaluated by a method described in examples to be described below (basic coloration test). The color saturation (b*) of the CIE color space (JIS Z 8729) when the product 1,3-butylene glycol of the present embodiment has been evaluated by the method is not particularly limited; however, for example, the average value of three times of measurement is preferably 4.0 or less, more preferably 3.8 or less and particularly preferably 3.5 or less.

### Examples

Hereinafter, the present invention will be more specifically described with examples, but the present invention is not limited to the following examples. As crude 1,3-butylene glycol that served as a raw material, acetaldol that had been subjected to hydrogen reduction, then, low-boiling fraction distillation and high-boiling fraction distillation (distillation for removing high-boiling components contained in the crude 1,3-butylene glycol) was used. A variety of analyses and evaluations were performed according to the following. In a gas chromatography analysis 2 under the following conditions, the peak area percentage of the crude 1,3-butylene glycol, which served as the raw material, was 99.6%.

### [Gas Chromatography Analysis 1]

Product 1,3-butylene glycols obtained in Examples 1 to 3 and Comparative Example 1 were subjected to gas chromatography analysis 1 according to the following method.

### (Conditions of Gas Chromatography Analysis 1)

Analysis device: 7890A gas chromatography system manufactured by Agilent Technologies, Inc.

Analysis column: DB-225 (length 30 m × inner diameter 0.25 mm × film thickness 0.25 µm, stationary phase: (50% cyanopropyl-phenyl) dimethylpolysiloxane) manufactured by Agilent Technologies, Inc.

Temperature rising conditions: The temperature was retained at 50°C for five minutes, then, raised from 50°C up to 135°C at 10 °C/minute, retained at 135°C for nine minutes, raised from 135°C up to 220°C at 15 °C/minute and retained at 220°C for 12 minutes.
Sample introduction temperature: 220°C
Carrier gas: Nitrogen
Gas flow rate in column: 0.5 mL/minute
Detector and detection temperature: Flame ionization detector (FID) and 220°C
Control mode: Constant flow
Split ratio: Splitless
Injection opening vent purge: 60 mL/minute
Purge start time; One minute
Sample injection condition: 0.6 µL

Sample preparation: n-Dodecane manufactured by GL Sciences Inc. was diluted with 2-propanol manufactured by Fujifilm Wako Pure Chemical Corporation to 24 ppm, thereby preparing an inner standard solution. 0.5 g of the product 1,3-butylene glycol and 0.1 g of the inner standard solution were mixed together, thereby preparing a sample.

Calculation of sum of n-dodecane equivalent concentrations: In a case where the relative retention time of n-dodecane that was detected under the above-described conditions was regarded as 1.00, the total area value of peaks that were detected at relative retention times of 0.80 to 0.99, excluding the peak of the acetal form between methyl ethyl ketone and 1,3-butylene glycol (the detected peak area value) was converted to the sum of the n-dodecane equivalent concentrations using the following formula (1), and this was regarded as an impurity concentration A. The total area value of the peak of 2-ethylcrotonaldehyde and the peak of the acetal form between n-butyraldehyde and 1,3-butylene glycol (the detected peak area value) was converted to the sum of the n-dodecane equivalent concentrations using the following formula (1), and this was regarded as an impurity concentration B. The sum of n-dodecane equivalent concentrations (ppm) = the n-dodecane concentration (ppm) × the detected peak area value/the area value of n-dodecane (1)

Here, the n-dodecane concentration (ppm) is a value that is calculated using the following formula (2). The n-dodecane concentration (ppm) = (the mass of n-dodecane in sample/the mass of product 1,3-butylene glycol in sample) × 106 (2)

### [Gas Chromatography Analysis 2]

The crude 1,3-butylene glycols that served as the raw material in Examples 1 to 3 and Comparative Example 1 and the product 1,3-butylene glycols obtained in Examples 1 to 3 and Comparative Example 1 were subjected to gas chromatography analysis 2 according to the following method.

### (Conditions of Gas Chromatography Analysis 2)

Analysis device: 7890B gas chromatography system manufactured by Agilent Technologies, Inc.

Analysis column: DB-WAX (column in which the stationary phase is polyethylene glycol; length 30 m × inner diameter 0.25 mm × film thickness 0.25 µm) manufactured by Agilent Technologies, Inc.

Temperature rising conditions: The temperature was raised from 80°C up to 230°C at 5 °C/minute and then retained at 230°C for 10 minutes.
Sample introduction temperature: 250°C
Carrier gas: Nitrogen
Gas flow rate in column: 0.5 mL/minute
Detector and detection temperature: Flame ionization detector (FID) and 250°C
Control mode: Constant flow
Split ratio: 50:1
Sample injection condition: 1 µL

### [Skin Sensitization Test]

The product 1,3-butylene glycols obtained in Examples 1 to 3 and Comparative Example 1 were evaluated for skin sensitization according to the following method.

### (Conditions of Skin Sensitization Test)

15 mg of cysteine-containing peptide for DPRA manufactured by Scrum Inc. and 30 mL of a 0.05 M phosphate buffer were mixed together, thereby preparing a 0.05 M phosphate buffer solution containing 0.667 mM of the peptide (hereinafter, peptide solution). After one hour elapsed from the preparation of the peptide solution, 750 µL of the peptide solution and 250 µL of acetonitrile were put into each of three brown sample bottles for HPLC, thereby preparing three reference solutions. After two hours elapsed from the preparation of the reference solutions, 750 µL of the peptide solution, 200 µL of acetonitrile and 50 µL of 1,3-butylene glycol were put into a separate brown sample bottle for HPLC, thereby preparing a specimen solution (specimen solution 1). Furthermore, after four hours elapsed and six hours elapsed from the preparation of the reference solutions, specimen solutions were prepared by the same operation (specimen solution 2 and specimen solution 3). Each of the three reference solutions and the specimen solutions (specimen solution 1, specimen solution 2 and specimen solution 3) was measured by an HPLC analysis after 72 ± 2 hours elapsed from the preparation of each solution, and the three-time average value of the peak heights of peptide in each of the reference solutions and the specimen solutions was calculated. A peptide reduction percentage was calculated from the calculated three-time average value using the following formula (3) and regarded as an evaluation result. Peptide reduction percentage (%) = (three-time average value of peak heights of peptide in specimen solution/three-time average value of peak heights of peptide in reference solution) × 100 (3)

In the present test, a specimen solution of the product 1,3-butylene glycol of Comparative Example 1 that served as a reference and a specimen solution of the product 1,3-butylene glycol of each of Examples 1 to 3 were each tested on the same day from the viewpoint of reproducibility.

### (Conditions of HPLC analysis in Skin Sensitization Test)

Analysis device: Agilent 1260 InfinityII manufactured by Agilent Technologies, Inc.
Detector: Agilent 1260 InfinityII UV-Vis detector G7114A manufactured by Agilent Technologies, Inc.
Detection wavelength: 220 nm
Analysis column: Zorbax SB-C-18 (particle diameter 3.5 µm, inner diameter × length = 2.1 mm × 10 mm) manufactured by Agilent Technologies, Inc.
Column temperature: 30°C
Measurement time: 20 min.
Mobile phases:
   A 0.1 vol% trifluoroacetic acid aqueous solution
   B 0.085 vol% trifluoroacetic acid-acetonitrile solution
Gradient:
   A/B = 90/10 to 75/25 (10 min.)
   A/B = 75/25 to 10/90 (1 min.)
   A/B = 10/90 (2 min.)
   A/B = 10/90 to 90/10 (0.5 min.)
   A/B = 90/10 (6.5 min.)
   Mobile phase flow rate: 0.35 mL/min.
   Sample injection condition: 5 µL

### [Basic Coloration Test]

The product 1,3-butylene glycols obtained in Examples 1 to 3 and Comparative Example 1 were evaluated for coloration under a basic condition according to the following method.

### (Conditions of Basic Coloration Test)

13 g of water and 2 g of potassium hydroxide were added to a 100 mL heat-resistant medium bottle and mixed together, and 6 g of the product 1,3-butylene glycol, which was a subject, was further added thereto. Next, the heat-resistant medium bottle was immersed in a water bath, and a heat treatment was performed at 90°C for one hour. After the solution that had been subjected to the heat treatment was cooled to room temperature, the color saturation (b*) of the solution was measured with a spectral colorimeter SE2000 manufactured by Nippon Denshoku Industries Co., Ltd. and regarded as a grade.

### [Example 1]

### (Hydrogenation Treatment Step)

The crude 1,3-butylene glycol and a hydrogen gas were circulated in a catalyst layer packed with 10 mL of a 0.5% Pd/C catalyst manufactured by N. E. Chemcat Corporation such that LHSV reached 8.0 h⁻¹ and GHSV reached 420 h⁻¹, respectively, and hydrogen reduction was performed (hydrogenation treatment). The hydrogen pressure during a reaction was 0.7 MPa, and the reaction temperature was set to 135°C.

As a result of the gas chromatography analysis 1 on the obtained product 1,3-butylene glycol, in a case where the relative retention time of n-dodecane was regarded as 1.00, the sum of n-dodecane equivalent concentrations of peaks that were detected at relative retention times of 0.80 to 0.99, excluding the peak of an acetal form between methyl ethyl ketone and 1,3-butylene glycol, was 21 ppm (impurity concentration A). In addition, the sum of n-dodecane equivalent concentrations of the peak of 2-ethylcrotonaldehyde and the peak of the acetal form between n-butyraldehyde and 1,3-butylene glycol was 12 ppm (impurity concentration B).

As a result of the gas chromatography analysis 2 on the product 1,3-butylene glycol, the area percentage of the peaks of the product 1,3-butylene glycol was 99.6%.

As a result of the skin sensitization test on the product 1,3-butylene glycol, the peptide reduction percentage was 45 in terms of a relative value in a case where the result of Comparative Example 1 was regarded as 100 as a reference. In addition, as a result of the basic coloration test, the color saturation (b*) was 2.8**.**

These results relating to the product 1.3-butylene glycol are shown in Table 1.

### [Example 2]

The steps, the analyses and the tests were performed in the same manner as in Example 1 except that the reaction temperature in the hydrogenation treatment step was set to 125°C.

As a result of the gas chromatography analysis 1 on the obtained product 1,3-butylene glycol, in a case where the relative retention time of n-dodecane was regarded as 1.00, the sum of n-dodecane equivalent concentrations of peaks that were detected at relative retention times of 0.80 to 0.99, excluding the peak of an acetal form between methyl ethyl ketone and 1,3-butylene glycol, was 18 ppm (impurity concentration A). In addition, the sum of n-dodecane equivalent concentrations of the peak of 2-ethylcrotonaldehyde and the peak of the acetal form between n-butyraldehyde and 1,3-butylene glycol was 10 ppm (impurity concentration B).

As a result of the gas chromatography analysis 2 on the product 1,3-butylene glycol, the area percentage of the peaks of the product 1,3-butylene glycol was 99.6%.

As a result of the skin sensitization test on the product 1,3-butylene glycol, the peptide reduction percentage was 43 in terms of a relative value in a case where the result of Comparative Example 1 was regarded as 100 as a reference. In addition, as a result of the basic coloration test, the color saturation (b*) was 3.2.

### [Example 3]

### (Heat treatment step)

The crude 1,3-butylene glycol was circulated in a tank heated to 100°C such that the residence time reached one hour to perform a heat treatment.

### (Hydrogenation Treatment Step)

The hydrogenation treatment step was performed in the same manner as in Example 1 except that 1,3-butylene glycol obtained in the heat treatment step was used as the raw material in the hydrogenation treatment step and the reaction temperature in the hydrogenation treatment step was set to 100°C.

### (Low-Boiling Fraction Distillation Step)

Low-boiling fraction distillation was performed at an oil bath temperature of 126°C and a pressure of 1.6 kPa in a distillation device equipped with a 500 mm-high packed tower (inner diameter: 22 mm) packed with 3 mm-sized Dixon rings such that the height of the 1,3-butylene glycol obtained in the hydrogenation treatment step packed reached 415 mm, a distillate of a weight fraction of 10% of the amount of liquid charged from the distillation device top was distilled away, and a product 1,3-butylene glycol was obtained from the distillation device bottom.

As a result of the gas chromatography analysis 1 on the obtained product 1,3-butylene glycol, in a case where the relative retention time of n-dodecane was regarded as 1.00, the sum of n-dodecane equivalent concentrations of peaks that were detected at relative retention times of 0.80 to 0.99, excluding the peak of an acetal form between methyl ethyl ketone and 1,3-butylene glycol, was 0 ppm (impurity concentration A). In addition, the sum of n-dodecane equivalent concentrations of the peak of 2-ethylcrotonaldehyde and the peak of the acetal form between n-butyraldehyde and 1,3-butylene glycol was 0 ppm (impurity concentration B).

As a result of the gas chromatography analysis 2 on the product 1,3-butylene glycol, the area percentage of the peaks of the product 1,3-butylene glycol was 99.7%.

As a result of the skin sensitization test on the product 1,3-butylene glycol, the peptide reduction percentage was 22 in terms of a relative value in a case where the result of Comparative Example 1 was regarded as 100 as a reference. In addition, as a result of the basic coloration test, the color saturation (b*) was 0.7.

### [Comparative Example 1]

The steps, the analyses and the tests were performed in the same manner as in Example 1 except that NiSAT340 (composition; NiO, SiO₂, Al₂O₃ and the like) manufactured by Clariant AG was used as the catalyst in the hydrogen reduction.

As a result of the gas chromatography analysis 1 on the obtained product 1,3-butylene glycol, in a case where the relative retention time of n-dodecane was regarded as 1.00, the sum of n-dodecane equivalent concentrations of peaks that detected at relative retention times of 0.80 to 0.99, excluding the peak of an acetal form between methyl ethyl ketone and 1,3-butylene glycol, was 28 ppm (impurity concentration A). In addition, the sum of n-dodecane equivalent concentrations of the peak of 2-ethylcrotonaldehyde and the peak of the acetal form between n-butyraldehyde and 1,3-butylene glycol was 27 ppm (impurity concentration B).

As a result of the gas chromatography analysis 2 for the product 1,3-butylene glycol, the area percentage of the peaks of the product 1,3-butylene glycol was 99.6%.

The skin sensitization test was performed for the product 1,3-butylene glycol, and the obtained peptide reduction percentage was regarded as 100 that served as a reference at the time of relative comparison with the examples. In addition, as a result of the basic coloration test, the color saturation (b*) was 19.6**.**

**[Table 1]**

| | | Example 1 | Example 2 | Example 3 | Comparative Example 1 |
|---|---|---|---|---|---|
| Gas chromatography analysis 1 | Impurity concentration A (ppm) | 21 | 18 | 0 | 28 |
| | Impurity concentration B (ppm) | 12 | 10 | 0 | 27 |
| Skin sensitization test | Relative value of peptide reduction percentage | 45 | 43 | 22 | 100 |
| Basic coloration test | Color saturation (b*) | 2.8 | 3.2 | 0.7 | 19.6 |

The present application is based on a Japanese patent application filed to the Japanese Patent Office on May 23, 2022 (Japanese Patent Application No. 2022-083950), the content of which is incorporated herein by reference.

### Industrial Applicability

1,3-Butylene glycol of the present invention is industrially available as a raw material of synthetic resins, a raw material of surfactants, a solvent, an antifreeze, a cosmetic raw material or the like.

## Claims

1. A product 1,3-butylene glycol, wherein, in a gas chromatography analysis by a splitless injection method under the following conditions, when a relative retention time of n-dodecane is regarded as 1.00, a sum of n-dodecane equivalent concentrations of peaks that appear within a relative retention time range of 0.80 to 0.99, excluding a peak of an acetal form between methyl ethyl ketone and 1,3-butylene glycol, is 25 mass ppm or less,
wherein the conditions of the gas chromatography analysis are as follows:
analysis column: a column in which a stationary phase is (50% cyanopropyl-phenyl) dimethylpolysiloxane (length 30 m × inner diameter 0.25 mm × film thickness 0.25 µm),
temperature rising conditions: a temperature is retained at 50°C for five minutes, then, raised from 50°C up to 135°C at 10 °C/minute, retained at 135°C for nine minutes, raised from 135°C up to 220°C at 15 °C/minute and retained at 220°C for 12 minutes,
sample introduction temperature: 220°C,
carrier gas: nitrogen,
gas flow rate in column: 0.5 mL/minute,
detector and detection temperature: flame ionization detector (FID) and 220°C,
control mode: constant flow,
split ratio: splitless,
injection opening vent purge: 60 mL/minute,
purge start time: one minute,
sample injection condition: 0.6 µL,
sample preparation: n-dodecane, which is an inner standard substance, is diluted with 2-propanol to 24 mass ppm to prepare an inner standard solution, and then 0.5 g of the product 1,3-butylene glycol and 0.1 g of the inner standard solution are mixed together, thereby preparing a sample, and
calculation of sum of n-dodecane equivalent concentrations: in a case where the relative retention time of n-dodecane that is detected under the gas chromatography analysis conditions is regarded as 1.00, a total area value of peaks that are detected within the relative retention time range of 0.80 to 0.99, excluding the peak of the acetal form between methyl ethyl ketone and 1,3-butylene glycol (a detected peak area value) is converted to the sum of the n-dodecane equivalent concentrations using the following formula (1), the sum of n-dodecane equivalent concentrations (mass ppm) = an n-dodecane concentration (mass ppm) × the detected peak area value/an area value of n-dodecane (1),
where the n-dodecane concentration (mass ppm) is a value that is calculated using the following formula (2), the n-dodecane concentration (mass ppm) = (a mass of n-dodecane in sample/a mass of product 1,3-butylene glycol in sample) × 106 (2).

2. A product 1,3-butylene glycol, wherein, in a gas chromatography analysis by a splitless injection method under the following conditions, a sum of n-dodecane equivalent concentrations of a peak of 2-ethylcrotonaldehyde and a peak of an acetal form between n-butyraldehyde and 1,3-butylene glycol is 20 mass ppm or less,
wherein the conditions of the gas chromatography analysis are as follows:
analysis column: a column in which a stationary phase is (50% cyanopropyl-phenyl) dimethylpolysiloxane (length 30 m × inner diameter 0.25 mm × film thickness 0.25 µm),
temperature rising conditions: a temperature is retained at 50°C for five minutes, then, raised from 50°C up to 135°C at 10 °C/minute, retained at 135°C for nine minutes, raised from 135°C up to 220°C at 15 °C/minute and retained at 220°C for 12 minutes,
sample introduction temperature: 220°C,
carrier gas: nitrogen,
gas flow rate in column: 0.5 mL/minute,
detector and detection temperature: flame ionization detector (FID) and 220°C,
control mode: constant flow,
split ratio: splitless,
injection opening vent purge: 60 mL/minute,
purge start time: one minute,
sample injection condition: 0.6 µL,
sample preparation: n-dodecane, which is an inner standard substance, is diluted with 2-propanol to 24 mass ppm to prepare an inner standard solution, and then 0.5 g of the product 1,3-butylene glycol and 0.1 g of the inner standard solution are mixed together, thereby preparing a sample, and
calculation of sum of n-dodecane equivalent concentrations: a total area value of the peak of 2-ethylcrotonaldehyde and the peak of the acetal form between n-butyraldehyde and 1,3-butylene glycol that are detected under the gas chromatography analysis condition (a detected peak area value) is converted to the sum of the n-dodecane equivalent concentrations using the following formula (1), the sum of n-dodecane equivalent concentrations (mass ppm) = an n-dodecane concentration (mass ppm) × the detected peak area value/an area value of n-dodecane
where the n-dodecane concentration (mass ppm) is a value that is calculated using the following formula (2), the n-dodecane concentration (mass ppm) = (a mass of n-dodecane in sample/a mass of product 1,3-butylene glycol in sample) × 106

3. The product 1,3-butylene glycol according to claim 1 or 2, wherein, in the gas chromatography analysis under the following conditions, an area percentage of a peak of 1,3-butylene glycol is 99.5% or more,
wherein the conditions of the gas chromatography analysis are as follows:
analysis column: a column in which a stationary phase is polyethylene glycol (length 30 m × inner diameter 0.25 mm × film thickness 0.25 µm),
temperature rising conditions: the temperature was raised from 80°C up to 230°C at 5 °C/minute and then retained at 230°C for 10 minutes,
sample introduction temperature: 250°C,
carrier gas: nitrogen,
gas flow rate in column: 0.5 mL/minute,
detector and detection temperature: flame ionization detector (FID) and 250°C,
control mode: constant flow,
split ratio: 50:1,
sample injection condition: 1 µL.
